# EUROPÄISCHE PATENTSCHRIFT

(11) **EP 1 017 783 B1**
(45) Veröffentlichungstag und Bekanntmachung des Hinweises auf die Patenterteilung: **28.01.2004**
(21) Anmeldenummer: 98961099.3
(22) Anmeldetag: 14.10.1998
(51) Int. Cl.: C12M 1/107, C12M 1/08

(54) **FAULBEHÄLTER MIT RÜHRWERK UND VERFAHREN ZUM BETREIBEN EINES RÜHRWERKS IN EINEM FAULBEHÄLTER**
DIGESTER WITH AGITATOR AND METHOD FOR OPERATING AN AGITATOR IN A DIGESTER
DIGESTEUR A AGITATEUR ET PROCEDE POUR FAIRE FONCTIONNER UN AGITATEUR DANS UN DIGESTEUR

(30) Priorität: 18.12.1997 DE 19756485; 16.03.1998 DE 19811398
(43) Veröffentlichungstag der Anmeldung: 12.07.2000
(73) Patentinhaber: Lipp, Xaver, D-73479 Ellwangen (DE)
(72) Erfinder: Lipp, Xaver, D-73479 Ellwangen (DE)
(74) Vertreter: Patentanwälte Bartels und Partner
(86) Internationale Anmeldenummer: PCT/EP1998/006515
(87) Internationale Veröffentlichungsnummer: WO 1999/032600

(56) Entgegenhaltungen:
- WO-A-82/00299
- DE-A- 4 302 740
- DE-C- 19 621 914
- GB-A- 687 296
- SU-A- 181 039
- US-A- 3 635 796
- DATABASE WPI Section Ch, Week 8506 Derwent Publications Ltd., London, GB; Class D15, AN 85-034924 XP002095690 & JP 59 228998 A (MATSUSHITA ELEC IND CO LTD), 22. Dezember 1984

## Beschreibung

Die Erfindung betrifft einen Faulbehälter mit einem Rührwerk gemäß dem Oberbegriff des Anspruchs 1 und ein zugehöriges Verfahren zum Betreiben eines Rührwerks in einem Faulbehälter.

Derartige Faulbehälter sind aus dem Einsatz in landwirtschaftlichen Biogasanlagen und kommunalen Kläranlagen bekannt. In dem Faulbehälter verweilen organische Abfallstoffe im Durchschnitt für mehrere Tage bei einer Temperatur, die über der Atmosphärentemperatur liegt und beispielsweise etwa 35°C beträgt. In dem Faulbehälter ist ein Rührwerk vorgesehen, das unter anderem zur Verteilung von über einen Füllstutzen eingeleiteten Abfallstoffen dient. Das Rührwerk wird in zeitlichen Abständen betrieben, damit ein optimaler Temperaturausgleich im Faulbehälter erfolgt und damit die neu eingeleiteten Abfallstoffe gleichmäßig im Faulbehälter verteilt werden. Das Rührwerk dient darüber hinaus dazu, die im Faulbehälter enthaltenen Bakterien gleichmäßig mit Nährstoffen zu versorgen, die Bildung von Schichten zu verhindern, insbesondere die sich fortwährend bildenden Schwimm- und Sinkschichten zu zerstören, und die Gasbläschen freizusetzen, die in tieferen Schichten an Schlammteilchen gebunden sind. Durch den Gärprozeß im Faulbehälter erfolgt eine Gasabscheidung, wobei das entstehende Biogas in einem Speicherbehälter speicherbar ist.

Die Erfindung geht im Oberbegriff von der WO 82/00299 aus, die einen kugel förmigen Faulbehälter mit einen Schraubenpumpe zeigt, die in einem zentrischen Schachtrohr angeordnet ist und eine vertikal verlaufende Antriebsachse aufweist. Das Schachtrohr ist nach oben aus dem Faulbehälter herausgeführt und umfaßt ein den oberen Teil des Schachtrohres gegen den Gasraum des Faulbehälters abdichtendes Schott. Auf der Höhe des Kugeläquators, der zugleich die Füllgrenze des Faulbehälters bildet, weist das Schachtrohr mehrere Durchbrüche auf, über die die Schraubenpumpe das zu pumpende Material ansaugt. In den oberen Teil des Schachtrohrs ist ein Befüllrohr eingebaut, über welches Material dem Faulbehälter zuführbar ist.

Eine Mischeinrichtung für flüssigkeitsgefüllte Behälter ist aus der DE 94 04 188 U1 bekannt. Dabei wird eine mit einer Führungseinheit fest verbundene Umwälzeinrichtung mittels einer Hebevorrichtung in den Behälter entlang und außerhalb eines am Boden des Behälters befestigten Führungsrohrs abgesenkt und angehoben. Dabei ist der Abstand zwischen dem Führungsrohr und der Wand des Behälters so bemessen, daß die Umwälzeinrichtung die für ihre ungestörte Funktion notwendigen saugseitigen und radialen Freiräume besitzt.

Es ist Aufgabe der vorliegenden Erfindung, einen Faulbehälter mit einem Rührwerk sowie ein Verfahren zum Betreiben eines Rührwerks in einem Faulbehälter bereitzustellen, die eine bessere Durchmischung der Abfallstoffe im Faulbehälter bereitstellen und damit die Effizienz des Faulbehälters erhöhen. Dabei soll insbesondere durch eine verbesserte Durchmischung der Abfallstoffe die Gasabscheidung verbessert werden.

Die Aufgabe wird durch die in dem unabhängigen Patentanspruch offenbarte Vorrichtung und das zugehörige Verfahren gelöst. Besondere Ausführungsarten der Erfindung sind in den Unteransprüchen offenbart.

Die Aufgabe ist bei einem Faulbehälter gemäß dem Oberbegriff des Anspruchs 1 unter anderem dadurch gelöst, daß das Rührwerk in einem unterhalb des Füllstutzens angeordneten Rührschacht oder Rührrohr untergebracht ist. Außerdem schneidet die verlängerte Projektion der Antriebs- oder Rührachse den Umfang des Faulbehälters sekantenförmig, d.h. ist nicht auf den Mittelpunkt des Faulbehälters gerichtet.

Bei dem Faulbehälter handelt es sich in der Regel um einen zylindrischen, geschlossenen Behälter mit einer vorzugsweise kreisrunden Bodenfläche. Die Bodenfläche kann dabei grundsätzlich jede beliebige Kontur aufweisen. Vorteilhaft sind abgerundete Ecken oder die Kreisform, da diese das Entstehen einer Drehbewegung der Abfallstoffe im Faulbehälter fördern. Das Rührwerk weist neben einer Antriebsachse auch eine oder mehrere Rührschaufeln bzw. eine Rührschraube auf. Das Rührwerk ist dabei am Umfang bzw. am Rand des Faulbehälters angebracht. Über den Füllstutzen werden neue Abfallstoffe in den Faulbehälter eingegeben. Das Rührrohr verläuft in der Regel senkrecht und erstreckt sich vorzugsweise bis zu einem Abstand von 0,2 m bis 1 m unterhalb der jeweiligen Füllhöhe.

Das Rührwerk ist in der Regel in einem abgewinkelten, horizontalen Abschnitt des Rührrohres angeordnet, wobei die Antriebsachse vorzugsweise mittig und achsenparallel zu dem horizontalen Abschnitt des Rührrohres verläuft. Alternativ dazu kann das Rührwerk auch außerhalb des Rohrrohres, aber unmittelbar unterhalb dessen unteren Endes angeordnet sein. Dabei ist die Antriebsachse des Rührwerks nicht auf den Mittelpunkt des Faulbehälters gerichtet, sondern die verlängerte Antriebsachse schneidet den Umfang bzw. den Rand oder die Kontur des Faulbehälters sekantenförmig.

Dies hat den Vorteil, daß dadurch eine Dreh- bzw. Drehrührbewegung der Abfallstoffe in dem Faulbehälter erreicht wird. Dies führt zu einer besonders guten Durchmischung der Abfallstoffe und dadurch zu einer erhöhten Effizienz des Faulbehälters hinsichtlich der Gasentwicklung. Die Anordnung des Rührrohres unterhalb des Füllstutzens hat den Vorteil, daß dadurch neu eingefülltes Material unmittelbar in das Rührrohr angesaugt wird und damit besonders wirksam mit den im Faulbehälter befindlichen Abfallstoffen vermischt wird. Insbesondere wird durch diese erfindungsgemäße Anordnung des Rührrohres unterhalb des Füllstutzens die Ausbildung von Schwimmschichten durch neu eingeleitete Abfallstoffe wirksam vermieden.

Die besondere Ausführungsart der Erfindung gemäß Anspruch 2 hat den Vorteil, daß durch die Einhaltung eines gewissen Abstandes des Rührwerks vom Boden des Faulbehälters sich am Boden des Faulbehälters ein Bodensatz von ausgefaulten Abfallstoffen bilden kann, der durch die Rührbewegung nicht erneut aufgewirbelt wird. Dadurch wird eine unerwünschte Durchmischung der noch gasaktiven Abfallstoffe mit dem nicht mehr gasaktiven Bodensatz im Faulbehälter wirksam vermieden. Letztlich wird hierdurch weiterhin die Effizienz der Gasbildung im Faulbehälter erhöht.

Die besondere Ausführungsart gemäß dem Anspruch 3 hat den Vorteil, daß durch die dreieckförmige oder teilelliptische Ausbildung des Querschnittes des Rührrohres keine Behinderung der gewünschten Drehbewegung der Abfallstoffe durch das vorzugsweise am Umfang des Faulbehälters angebrachte Rührrohr erfolgt, so daß die Drehbewegung der Abfallstoffe besonders wirksam erfolgt. Alternativ zu einem dreieckförmig oder teilelliptischen Querschnitt kommt auch ein vieleckförmiger Querschnitt in Frage, der so ausgestaltet ist, daß er eine gleichmäßige Drehbewegung nicht behindert.

Die besondere Ausführungsart gemäß dem Anspruch 4 hat den Vorteil, daß durch die Höhenverstellbarkeit des Rührwerks in verschiedenen Höhen innerhalb des Faulbehälters gerührt werden kann, was zu einer besseren Durchmischung der Abfallstoffe und damit zu einer höheren Effizienz der Gasbildung führt. Die Höhenverstellung kann dabei entweder manuell oder motorisch sowie zusammen mit dem Rührwerk oder relativ dazu erfolgen.

Die besondere Ausführungsart gemäß dem Anspruch 5 hat den Vorteil, daß durch eine Verkippung der Antriebsachse aus der Horizontalen nicht nur eine horizontale Drehbewegung der Abfallstoffe bewirkt wird, sondern darüber hinaus auch eine Durchmischung der Abfallstoffe in vertikaler Richtung erfolgt. Der Winkel zwischen der Antriebsachse und der Horizontalen beträgt dabei vorzugsweise zwischen 5° und 45°.

Die besondere Ausführungsart gemäß Anspruch 6 hat den Vorteil, daß durch die nach außen geführte Antriebsachse die Motoreneinheit außerhalb des Faulbehälters angeordnet ist, was insbesondere hinsichtlich von Wartungsarbeiten an dem Motor von Vorteil ist. Darüber hinaus können durch das Vorsehen einer Getriebeverbindung verschiedene Über- bzw. Untersetzungsverhältnisse und damit verschiedene Drehzahlen des Rührwerks realisiert werden. Die Getriebeverbindung kann dabei auch über eine Zahnradverbindung erfolgen, vorzugsweise wird jedoch eine Ketten- oder Riemenverbindung vorgesehen. Darüber hinaus kann eine Kupplung, beispielsweise auch eine Rutschkupplung, vorgesehen sein.

Die besondere Ausführungsart gemäß dem Anspruch 7 hat den Vorteil, daß durch die Ausführung der Antriebsachse außerhalb des Faulbehälters als Zapfwelle zusätzlich zum Antrieb beispielsweise durch den Elektromotor ein Antrieb durch einen Verbrennungsmotor, beispielsweise durch einen Traktor, möglich ist. Dieses Rühren mittels eines Verbrennungsmotors erlaubt höhere Rührleistungen, beispielsweise zum Zwecke einer besonders gründlichen Durchmischung der Abfallstoffe in dem Faulbehälter oder zu Reinigungszwecken.

Die besondere Ausführungsart gemäß Anspruch 8 hat den Vorteil, daß die Länge des Rührrohres insbesondere an den Flüssigkeitsstand im Faulbehälter adaptierbar ist. Die Länge des Rührrohres ist dabei vorzugsweise so zu wählen, daß der Abstand vom oberen Ende des Rührrohres bis zur Oberfläche der Flüssigkeit im Faulbehälter auch bei veränderlichen Füllständen annähernd konstant ist. Dadurch kann unter Berücksichtigung der lichten Weite des Rührrohres und der Saugleistung des Rührwerkes eine optimale Ansaugwirkung auf die sich an der Oberfläche bildenden Schwimmschichten erreicht werden. Insbesondere kann der Abstand unter Berücksichtigung der Konsistenz und/oder Viskosität der Flüssigkeit so eingestellt werden, daß sich während des Rührvorganges die Flüssigkeitsoberfläche im Bereich des Rührrohres trichterförmig absenkt. Dadurch werden insbesondere auch Verklumpungen, die sich an der Oberfläche bilden, optimal angesaugt und durch das Rührwerk aufgelöst. Die Effizienz des Faulbehälters wird dadurch erhöht, insbesondere wird durch die verbesserte Durchmischung der Abfallstoffe die Gasabscheidung verbessert.

Die vorliegende Erfindung ist nicht auf Rührwerke für Faulbehälter beschränkt, sondern kann ebenso für andere Einsatzzwecke Verwendung finden, beispielsweise für den Einsatz in einem Behälter mit einem Rührwerk zur Lebensmittelherstellung oder -verarbeitung.

Das Rührrohr kann als ein- oder mehrteiliger hohlzylindrischer Körper ausgeführt sein, es kann aber auch durch formschlüssiges Zusammenwirken eines Bleches mit der Wandung des Faulbehälters derart gebildet werden, daß das Blech einen Teil der Wandung des Rührrohres bzw. Rührschachtes bildet und die Wandung des Faulbehälters den anderen Teil der Wandung des Rührrohres bildet.

Gemäß Anspruch 9 weist das Rührrohr mindestens zwei teleskopartig ineinander verschiebbare Teilstücke auf. Der Einsatz von teleskopartigen Teilstücken oder Teilrohren erlaubt, abhängig von der Anzahl der eingesetzten Teilstücke, eine große Längenvarianz.

Gemäß Anspruch 10 weist das obere Teilrohr eine stabartige Verlängerung auf, die über den oberen Rand des oberen Teilrohres in die Höhe ragt. Diese Verlängerung ragt vorzugsweise aus der Oberfläche der in dem Behälter befindlichen Flüssigkeit heraus und erlaubt dem Betreiber des Faulbehälters auch bei stark getrübten oder undurchsichtigen Flüssigkeiten die exakte Bestimmung des Abstandes des oberen Randes des Rührrohres von der Flüssigkeitsoberfläche. Als ein günstiger Wert für diesen Abstand hat sich ein Bereich von 0,1 m bis 1,0 m erwiesen, vorzugsweise zwischen 0,15 m und 0,30 m. Eine Alternative oder Ergänzung für die Abstandsbestimmung sind Ultraschallmessungen, insbesondere im Klärwerksbereich.

Gemäß Anspruch 11 sind die Teilrohre mit Klemm- oder Rastmitteln zur Fixierung der einzelnen Teilrohre untereinander versehen. Dadurch wird eine sichere und gegebenenfalls auch stufenlose Längenadaption des Rührrohres erreicht.

Gemäß Anspruch 12 ist ein Teilrohr, vorzugsweise das obere Teilrohr, mechanisch mit einem Schwimmer gekoppelt, wobei der Schwimmer auf der Oberfläche der Flüssigkeit schwimmt. Durch die geometrische Ausgestaltung der mechanischen Kopplung zwischen Schwimmer und Teilrohr ist ein konstanter Abstand zwischen dem oberen Ende des Rührrohres und der Flüssigkeitsoberfläche gewährleistet. Die mechanische Kopplung kann beispielsweise durch eine Aluminiumstange realisiert werden, die so ausgerichtet ist, daß sie mit der Horizontalen einen Winkel von beispielsweise 30° einschließt und vom Rührrohr ausgehend radial auf den Mittelpunkt des Füllbehälters gerichtet ist. Alternativ können auch mehrere Schwimmkörper an einer oder mehreren Stangen angeordnet sein.

Die zugrundeliegende Aufgabe wird auch durch ein Verfahren gemäß Anspruch 13 zum Betreiben eines Faulbehälters gelöst. Durch das Rühren des Inhalts des Faulbehälters, während neues Abfallmaterial über den Einfüllstutzen in den Faulbehälter eingefüllt wird, wird eine gleichmäßige Durchmischung von neuen und bereits im Faulbehälter vorhandenen Abfallstoffen erreicht. Dies gilt insbesondere aufgrund der Ausgestaltung des Faulbehälters gemäß Anspruch 1, wonach das Rührrohr bzw. der Rührschacht unterhalb des Füllstutzens angeordnet ist. Dadurch wird eine Homogenisierung der im Faulbehälter befindlichen Abfallstoffe erzielt und damit letztlich die Effizienz der Gasbildung erhöht.

Die besondere Ausführungsart der Erfindung gemäß dem Anspruch 14 hat den Vorteil, daß durch das Rühren in umgekehrter Drehrichtung die Strömungsrichtung im Rührrohr umgekehrt wird und dadurch das Rührrohr gereinigt wird, insbesondere wird dadurch Fasermaterial gelöst und abgespült, . das sich an dem Ende des Saugrohrs, das im Normalbetrieb die Ansaugseite des Rührrohres bildet, abgesetzt hat.

Weitere Vorteile, Merkmale und Einzelheiten der Erfindung ergeben sich aus der nachfolgenden Beschreibung, in der unter Bezugnahme auf die Zeichnungen ein Ausführungsbeispiel im einzelnen beschrieben wird. Dabei können die in den Ansprüchen und in der Beschreibung erwähnten Merkmale jeweils einzeln für sich oder in beliebiger Kombination erfindungswesentlich sein.
- Fig. 1: zeigt eine Seitenansicht eines erfindungsgemäßen Faulbehälters im Querschnitt;
- Fig. 2: zeigt eine Draufsicht auf den Faulbehälter der Fig. 1;
- Fig. 3: zeigt einen vergrößerten Ausschnitt eines Faulbehälters ähnlich dem der Fig. 1 mit einem teleskopartig ausgebildeten Rührrohr;
- Fig. 4: zeigt ausschnittsweise die Draufsicht auf den Faulbehälter der Fig. 3

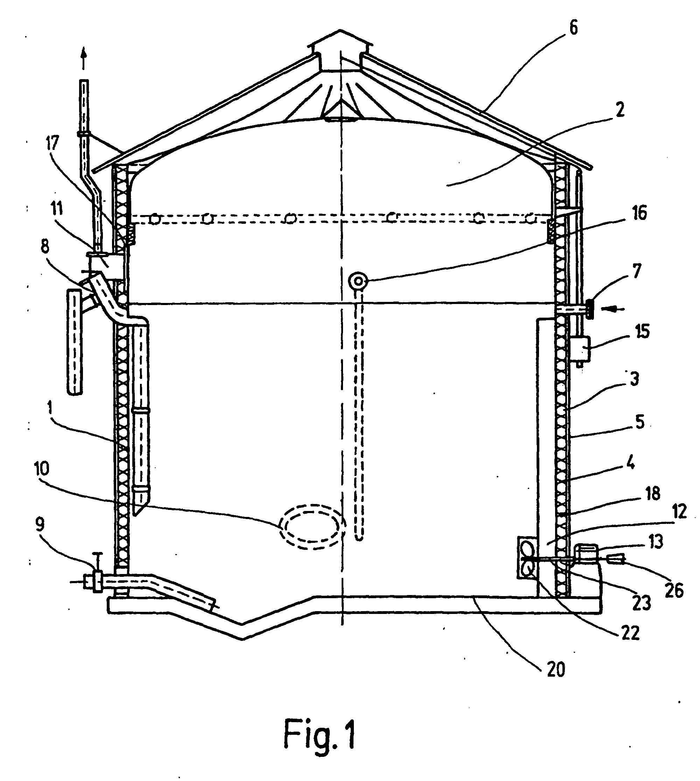

Die Fig. 1 zeigt eine Seitenansicht des erfindungsgemäßen Faulbehälters 1. Im dargestellten Beispiel bildet der Faulbehälter 1 einen Biogasreaktor mit integrierter Gasspeicherhaube 2. Der Faulbehälter 1 bildet einen zylindrischen, geschlossenen Raum mit kreisrunder Bodenfläche 20 und ist mit einem Dach 6 versehen. Umfänglich weist der Faulbehälter 1 eine Isolierung 3, eine Wandheizung 4 sowie eine Verkleidung 5 auf. Über den Füllstutzen 7 erfolgt der Zulauf von frischen Substraten bzw. von frischen Abfallstoffen. Der Ablauf 8 weist einen integrierten Notablauf auf. Über den absperrbaren Anschluß 9 erfolgt die Entleerung des Faulbehälters 1. Der Faulbehälter 1 weist darüber hinaus ein Mannloch 10 auf. Eine Vorrichtung 11 sorgt für eine Über- und Unterdrucksicherung der Gasspeicherhaube 2.

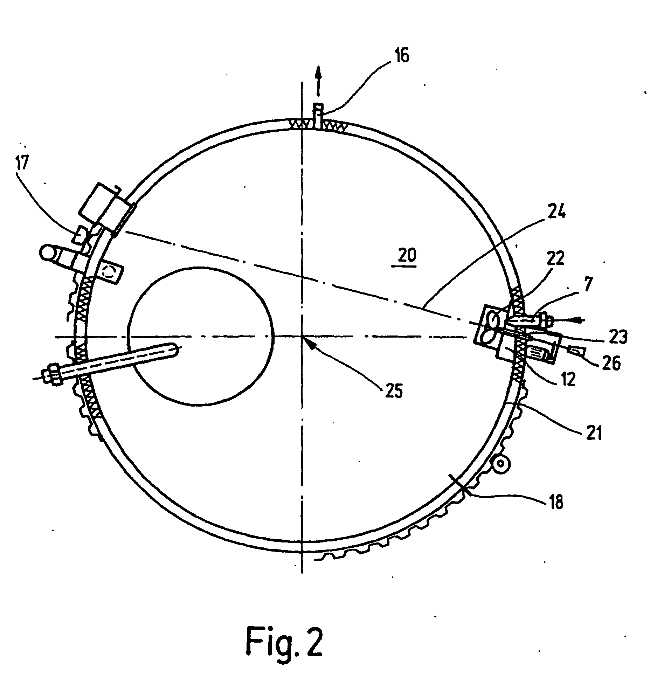

Am Umfang bzw. Rand 21 (Fig. 2) des Faulbehälters 1 ist ein Rührwerk 22 mit einer Antriebsachse 23 angeordnet, die von einem Antrieb 13 zur Homogenisierung der im Faulbehälter 1 befindlichen Abfallstoffe antreibbar ist. Der Antrieb 13 wird von einem Elektromotor gebildet, der über eine Getriebeverbindung, bestehend aus Kettenrädern und einer Antriebskette, mit der Antriebsachse 23 gekoppelt ist. Das Rührwerk 22 besteht aus einer mehrschaufligen Rührschraube.

Das Rührwerk 22 befindet sich in einem horizontalen Abschnitt des in seiner Längserstreckung vertikal angeordneten Rührrohres 12, das am Rand 21 des Faulbehälters 1 angeordnet ist. Das Rührrohr 12 erstreckt sich dabei bis zu einem Abstand von 0,2 m bis 1 m unterhalb der Füllhöhe der Abfallstoffe im Faulbehälter 1. Die lichte Weite bzw. der Durchmesser des Rührrohres 12 kann zwischen 0,5 m und 1,5 m betragen.

Die Antriebsachse 23 verläuft horizontal, kann jedoch in einer alternativen Ausführungsform auch gegenüber der Horizontalen um einen Winkel zwischen 0° und 90°, vorzugsweise um einem Winkel zwischen 5° und 45°, verkippt sein. Die Verkippung kann einstellbar sein.

Das Rührrohr 12 steht auf der Bodenfläche 20 des Faulbehälters 1 auf, es kann jedoch auch von der Bodenfläche 20 beabstandet enden. Das Rührwerk 22 ist vom Boden des Faulbehälters beabstandet, beispielsweise weist es einen Abstand zwischen 0,5 m und 1 m vom Boden 20 des Faulbehälters 1 auf.

Der Faulbehälter 1 weist darüber hinaus eine Gasinhaltsanzeige 15 sowie einen Anschluß 16 zur Gasentnahme auf. Weiterhin ist ein Anschluß 17 für eine Entschwefelung und ein Anschluß 18 zur Temperaturmessung der im Faulbehälter 1 befindlichen Abfallstoffe vorgesehen.

Die Fig. 2 zeigt eine Draufsicht auf den Faulbehälter 1 der Fig. 1.
Die verlängerte Projektion 24 der Antriebsachse 23 in die horizontale Ebene der Bodenfläche 20 schneidet den Umfang 21 des Faulbehälters 1 sekantenförmig. Die Antriebsachse 23 ist daher nicht auf den Mittelpunkt 25 des Faulbehälters 1 gerichtet. Das Rührrohr 12 ist dreieckförmig ausgebildet, wobei zwei Schenkel des Dreiecks einen rechten Winkel bilden und die "Hypotenuse" bzw. die dritte Seite durch einen Abschnitt des Umfangs 21 des Faulbehälters 1 gebildet wird. Das Rührwerk 22 ist in einem horizontalen Abschnitt des Rührrohres 12 untergebracht und ist unmittelbar unter dem Füllstutzen 7 angeordnet.

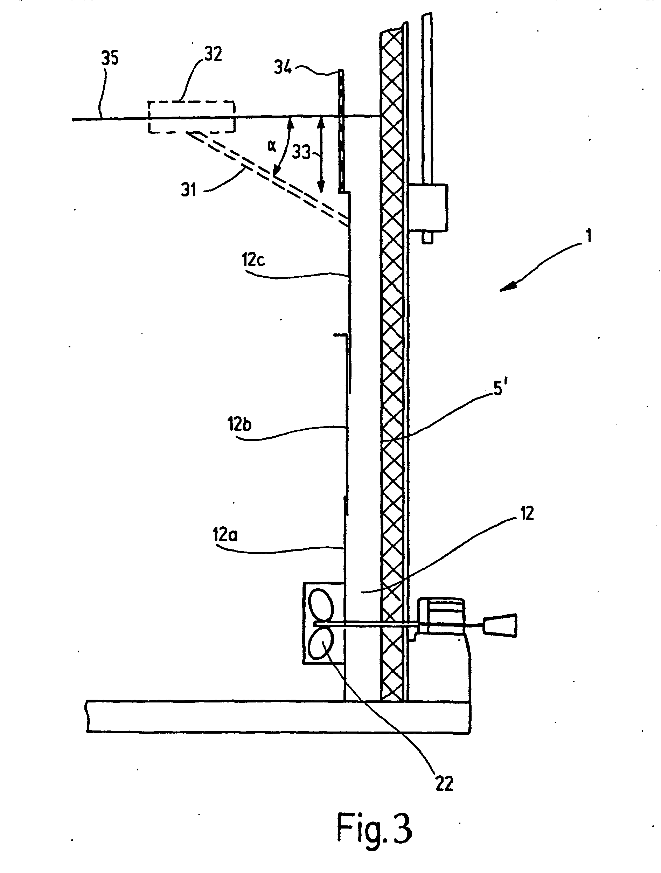

Die Fig. 3 zeigt einen vergrößerten Ausschnitt eines Faulbehälters ähnlich dem der Figur 1 mit einem teleskopartig ausgebildeten Rührrohr 12. Das Rührrohr 12 wird im vorliegenden Fall zu einem Teil durch die Wandung 5' des Faulbehälters 1 sowie durch drei Teilstücke 12a, 12b und 12c gebildet, die teleskopartig ineinander verschiebbar sind. Am oberen Teilrohr 12c ist über eine Stange 31 ein Schwimmer 32 befestigt. Die Stange 31 bildet mit der Flüssigkeitsoberfläche 35 einen Winkel *a* von etwa 30°. Durch die geometrische Ausgestaltung der mechanischen Kopplung des oberen Teilrohres 12c mit dem Schwimmer 32 mittels der Stange 31 wird ein konstanter Abstand 33 zwischen dem oberen Teilrohr 12c und der Flüssigkeitsoberfläche 35 gewährleistet. Dieser Abstand 33 beträgt vorzugsweise zwischen 0,15 m und 0,30 m.

Im Gegensatz zu der Fig. 1 ist in dem Ausschnitt der Figur 3 der Füllstutzen 7 nicht dargestellt. Der Füllstutzen 7 kann beispielsweise auch an einer beliebigen Stelle des Faulbehälters 1 angeordnet sein. Nach wie vor ist das Rührwerk 22 in bezug auf die vertikale Anordnung unterhalb des Füllstutzens 7 angeordnet. Auch der obere Rand des oberen Teilrohres 12c liegt vorzugsweise unterhalb des Füllstutzens 7.

Durch die Verwendung des Schwimmers 32 sind im dargestellten Beispiel keine Klemm- und Rastmittel erforderlich. Alternativ zum Schwimmer 32, oder auch zusätzlich dazu, können handelsübliche lösbare Klemm- oder Rastmittel zur Fixierung der Länge des Rührrohres 12 vorgesehen sein. Alternativ oder zusätzlich zum Schwimmer 32 kann auch eine stabartige Verlängerung 34 am oberen Teilrohr 12c angebracht sein, die teilweise aus der Flüssigkeitsoberfläche 35 herausragt, und dem Betreiber des Faulbehälters auch bei trüben oder undurchsichtigen Flüssigkeiten zuverlässig den Abstand 33 des oberen Teilrohre 12c von der Flüssigkeitsoberfläche 35 anzeigt.

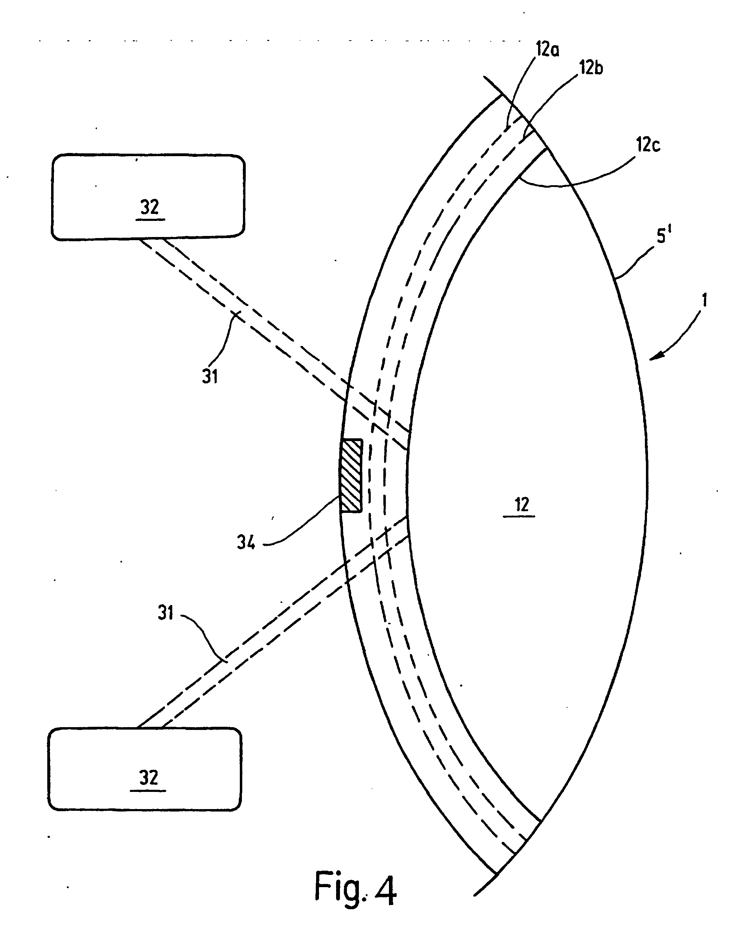

Die Fig. 4 zeigt ausschnittsweise die Draufsicht auf den Faulbehälter 1 der Fig. 3. Im dargestellten Beispiel sind zwei Schwimmkörper 32 an zwei Stangen 31 am oberen Teilrohr 12c befestigt. In der Draufsicht bilden die beiden Stangen 31 in der Horizontalebene einen Winkel von etwa 90°. Durch diese Anordnung der Schwimmkörper 32 wird die Ansaugung von Schwimmschichten sowohl entlang des Umfanges des Faulbehälters 1 als auch von der Mitte des Faulbehälters 1 nicht beeinträchtigt. Das Rührrohr 12 wird im vorliegenden Fall zum einen Teil von der Wandung 5' des Faulbehälters 1 und zum anderen Teil von den teilelliptischen Teilstücken 12a, 12b und 12c gebildet. Erkennbar ist in der Draufsicht auch die senkrecht zur Zeichenebene verlaufende stabartige Verlängerung 34.

## Patentansprüche

1. Faulbehälter (1) mit einem Rührwerk (22), das in einem Rührschacht oder in einem Rührrohr (12) angeordnet ist und eine Antriebsachse (23) aufweist, **dadurch gekennzeichnet,**
- **daß** der Faulbehälter eine vorzugsweise runde Bodenfläche aufweist,
- **daß** das Rührwerk (22) am Umfang (21) des Faulbehälters (1) angeordnet ist,
- **daß** die verlängerte Projektion (24) der Antriebsachse (23) in die horizontale Ebene den Umfang (21) sekantenförmig schneidet,
- und **daß** der Rührschacht oder das Rührrohr (12) unterhalb eines Einfüllstutzens (7) des Faulbehälters (1) angeordnet ist.

2. Faulbehälter (1) nach Anspruch 1, **dadurch gekennzeichnet, daß** das Rührwerk (22) von der Bodenfläche (20) des Faulbehälters (1) beabstandet ist.

3. Faulbehälter (1) nach Ansprüche 1 oder 2, **dadurch gekennzeichnet, daß** das Rührrohr (12) einen dreieckförmigen oder teilelliptischen Querschnitt aufweist.

4. Faulbehälter (1) nach einem derAnsprüche 1 bis 3, **dadurch gekennzeichnet, daß** das Rührwerk (22) in der Höhe innerhalb des Faulbehälters (1) mit einem Seil- oder Kettenzug verstellbar ist.

5. Faulbehälter (1) nach einem der Ansprüche 1 bis 4, **dadurch gekennzeichnet, daß** die Antriebsachse (23) mit der Horizontalen einen Winkel von 0° bis 90° bildet.

6. Faulbehälter (1) nach einem der Ansprüche 1 bis 5, **dadurch gekennzeichnet, daß** die Antriebsachse (23) nach außerhalb des Faulbehälters (1) geführt ist und über eine Getriebeverbindung vorzugsweise mit einem Elektromotor (13) antreibbar ist.

7. Faulbehälter (1) nach Anspruch 6, **dadurch gekennzeichnet, daß** die Antriebsachse (23) außerhalb des Faulbehälters (1) als Zapfwelle (26) ausgeführt ist.

8. Faulbehälter (1) nach einem der Ansprüche 1 bis 7, **dadurch gekennzeichnet, daß** das Rührrohr (12) in seiner vertikalen Ausdehnung veränderbar ist.

9. Faulbehälter (1) nach einem der Ansprüche 1 bis 8, **dadurch gekennzeichnet, daß** das Rührrohr (12) mindestens zwei teleskopartig ineinander verschiebbare Teilstücke (12a, 12b, 12c) aufweist.

10. Faulbehälter (1) nach Anspruch 9, **dadurch gekennzeichnet, daß** das obere Teilrohr (12c) an seinem oberen Ende eine nach oben gerichtete stabartige Verlängerung (34) mit einer Länge zwischen 0,1 m und 1,0 m aufweist, vorzugsweise zwischen 0,15 m und 0,30 m.

11. Faulbehälter (1) nach Anspruch 9 oder 10, **dadurch gekennzeichnet, daß** die Teilrohre (12a, 12b, 12c) mit Klemm- oder Rastmitteln versehen sind.

12. Faulbehälter (1) nach einem der Ansprüche 9 bis 11, **dadurch gekennzeichnet, daß** ein Teilrohr, vorzugsweise das obere Teilrohr (12c), mechanisch mit einem Schwimmer 32 gekoppelt ist.

13. Verfahren zum Betreiben eines Faulbehälters nach einem der Ansprüche 1 bis 12, wobei der Inhalt des Faulbehälters (1) in zeitlichen Abständen gerührt wird, **dadurch gekennzeichnet, daß** der Inhalt gerührt wird, während Material über einen Einfüllstutzen (7) in den Faulbehälter (1) eingefüllt wird.

14. Verfahren nach Anspruch 13, **gekennzeichnet durch** Rühren in umgekehrter Drehrichtung zur Reinigung des Rührrohres (12), beispielsweise in Zeitabstanden von einem Tag bis eine Woche.

## Claims

1. A fouling container (1) with a stirring mechanism (22), whereby the same is located within a stirring shaft or within a stirring pipe (12), and whereby the same incorporates a drive shaft (23), **characterised in that**
- the fouling container incorporates a preferably round floor area,
- the stirring mechanism (22) is located around the circumference (21) of the fouling container (1),
- that the extended projection (24) of the drive shaft (23) intersects the circumference (21) in a secant-shaped manner at a horizontal level,
- that the stirring shaft or the stirring pipe (12) is located below a filler pipe (7) of the fouling container (1).

2. A fouling container (1) according to Claim 1, **characterised in that** the stirring mechanism (22) is located at a distance from the floor area (20) of the fouling container (1).

3. A fouling container (a) according to Claim 1 or 2, **characterised in that** the stirring pipe (12) incorporates a triangular or partly elliptical cross section.

4. A fouling container (1) according to one of the preceding Claims 1 to 3, **characterised in that** the stirring mechanism (22) is movably adjustable with the aid of a rope or chain pulley at a certain height within the fouling container (1).

5. A fouling container (1) according to one of the preceding Claims 1 to 4, **characterised in that** the drive shaft (23) forms an angle of 0° to 90° together with the horizontal.

6. A fouling container (1) according to one of the preceding Claims 1 to 5, **characterised in that** the drive shaft (23) is routed along the outside of the said fouling container (1) and is further drivable via a gearbox connection, preferably with the aid of an electric motor (13).

7. A fouling container (1) according to Claim 6, **characterised in that** the drive shaft (23) is routed outside of the said fouling container (1) in the form of a spigot shaft (26).

8. A fouling container (1) according to one of the preceding claims 1 to 7, **characterised in that** the vertical expansion of the stirring pipe (12) is adjustable.

9. A fouling container (1) according to one of the preceding Claims 1 to 8, **characterised in that** the stirring pipe (12) incorporates at least two telescopically retractable part sections (12a, 12b, 12c).

10. A fouling container (1) according to Claim 9, **characterised in that** the upper pipe part section (12c) incorporates an upwardly orientated rod-like extension (34) with a length of between 0,1m and 1,0m, and preferably of between 0,15m and 0,30m, at its upper end.

11. A fouling container (1) according to Claim 9 or 10, **characterised in that** the pipe part sections (12a, 12b, 12c) are equipped with clip or engaging mechanisms.

12. A fouling container (1) according to one of the preceding Claims 9 to 11, **characterised in that** a pipe part section, preferably the upper pipe part section (12c), is mechanically coupled with a float (32).

13. A method for the operation of a fouling container (1) according to one of the preceding Claims 1 to 12, whereby the contents of the said fouling container (1) are stirred at regularly timed intervals, **characterised in that** the contents are stirred whilst material is added into the said fouling container (1) via a filler pipe (7).

14. A method according to Claim 13, **characterised in that** the stirring direction is reversed for the cleaning of the stirring pipe (12), for example at timed intervals of one day per week.

## Revendications

1. Récipient de putréfaction (1) avec un mélangeur (22) qui est disposé dans une cuve de mélange ou dans un tube de mélange (12) et comprend un axe d'entraînement (23),
**caractérisé en ce que**,
- le récipient de putréfaction présente un encombrement de préférence circulaire,
- le mélangeur (22) est disposé sur la périphérie (21) du récipient de putréfaction (1),
- la projection (24) allongée de l'axe d'entraînement (23) coupe en forme de sécante dans le plan horizontal la périphérie (21),
- et la cuve de mélange ou le tube de mélange (12) est disposé en dessous d'une tubulure de remplissage (7) du récipient de putréfaction (1).

2. Récipient de putréfaction (1) selon la revendication 1, **caractérisé en ce que** le mélangeur (22) est séparé de la surface (20) du récipient de putréfaction (1).

3. Récipient de putréfaction (1) selon la revendication 1 ou 2, **caractérisé en ce que** le tube de mélange (12) comprend une section transversale triangulaire ou partiellement elliptique.

4. Récipient de putréfaction (1) selon l'une quelconque des revendications 1 à 3, **caractérisé en ce que** le mélangeur (22) est réglable en hauteur à l'intérieur du récipient de putréfaction (1) à l'aide d'une commande par câble ou d'une tension de chaîne.

5. Récipient de putréfaction (1) selon l'une quelconque des revendications 1 à 4, **caractérisé en ce que** l'axe d'entraînement (23) forme avec le plan horizontal un angle de 0° à 90°.

6. Récipient de putréfaction (1) selon l'une quelconque des revendications 1 à 5, **caractérisé en ce que** l'axe d'entraînement (23) est dirigé vers l'extérieur du récipient de putréfaction (1) et peut être entraîné via une liaison de transmission de préférence avec un moteur électrique (13).

7. Récipient de putréfaction (1) selon la revendication 6, **caractérisé en ce que** l'axe d'entraînement (23) est conduit hors du récipient de putréfaction (1) en tant que prise de force (26).

8. Récipient de putréfaction (1) selon l'une quelconque des revendications 1 à 7, **caractérisé en ce que** le tube de mélange (12) peut être modifié dans son extension verticale.

9. Récipient de putréfaction (1) selon l'une quelconque des revendications 1 à 8, **caractérisé en ce que** le tube de mélange (12) comprend au moins deux tubes partiels (12a, 12b, 12c) pouvant être glissés l'un dans l'autre de façon télescopique.

10. Récipient de putréfaction (1) selon la revendication 9, **caractérisé en ce que** le tube partiel supérieur (12c) comprend sur son extrémité supérieure un prolongement (34) en forme de bâton orienté vers la partie supérieure d'une longueur comprise entre 0,1 m et 1,0 m, de préférence entre 0,15 m et 0,30 m.

11. Récipient de putréfaction (1) selon la revendication 9 ou 10, **caractérisé en ce que** les tubes partiels (12a, 12b, 12c) sont dotés de moyens de fixation ou d'encliquetage.

12. Récipient de putréfaction (1) selon l'une quelconque des revendications 9 à 11, **caractérise en ce qu'**un tube partiel, de préférence le tube partiel supérieur (12c), est couplé mécaniquement à un flotteur 32.

13. Procédé d'actionnement d'un récipient de putréfaction selon l'une quelconque des revendications 1 à 12, le contenu du récipient de putréfaction (1) étant mélangé à des intervalles de temps, **caractérisé en ce que** le contenu est mélangé pendant le remplissage de la matière par la tubulure de remplissage (7) dans le récipient de putréfaction (1).

14. Procédé selon la revendication 13, **caractérisé par** le mélange en sens inverse de rotation pour le nettoyage du tube de mélange (12) par exemple à des intervalles de temps allant d'un jour à une semaine.
